(19) Europäisches Patentamt
European Patent Office
Office européen des brevets

(11) Publication number: **0 028 515**
**B1**

(12) **EUROPEAN PATENT SPECIFICATION**

(45) Date of publication of patent specification: **21.03.84**

(51) Int. Cl.³: **C 07 C 69/16**, C 07 C 67/37, C 07 C 67/36

(21) Application number: **80303882.7**

(22) Date of filing: **31.10.80**

(54) **Process for producing ethylidenediacetate.**

(30) Priority: **02.11.79 JP 142544/79**
**06.11.79 JP 143676/79**

(43) Date of publication of application:
**13.05.81 Bulletin 81/19**

(45) Publication of the grant of the patent:
**21.03.84 Bulletin 84/12**

(84) Designated Contracting States:
**DE FR GB IT**

(56) References cited:
**EP - A - 0 025 702**
**FR - A - 2 303 788**
**FR - A - 2 404 618**

(73) Proprietor: **MITSUBISHI GAS CHEMICAL COMPANY, INC.**
**5-2, Marunouchi 2-chome Chiyoda-Ku**
**Tokyo (JP)**

(72) Inventor: **Isshiki, Tomiya**
**14-11, Umegaoka 2-Chome Setagaya-Ku**
**Toyko (JP)**
Inventor: **Kijima, Yasuhiko**
**641, Koda, Matsudo-shi**
**Chiba-ken (JP)**
Inventor: **Kondoh, Takao**
**61-6, Kikunodai 2-chome**
**Chufo-shi, Tokyo (JP)**

(74) Representative: **Shipton, Gordon Owen et al,**
**W.P. THOMPSON & CO. Coopers Building Church Street**
**Liverpool L1 3AB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 028 515 B1**

# Process for producing ethylidenediacetate

This invention relates to a process for producing ethylidenediacetate which comprises reacting carbon monoxide with at least one compound or mixture of two compounds selected from (1) dimethyl acetal, (2) acetaldehyde and methyl acetate, and (3) acetaldehyde and dimethyl ether.

In the prior art, ethylidenediacetate was synthesized from acetylene and acetic acid or by reacting acetaldehyde with acetic anhydride.

Recently, a process for producing ethylidenediacetate by reacting methyl acetate or dimethyl ether with carbon monoxide and hydrogen in the presence of a catalyst was proposed (refer to FR—A—2303788). In the process disclosed in this Patent Publication, ethylidenediacetate was synthesized from methyl acetate or dimethyl ether in the presence of a catalyst comprising rhodium or palladium as a main component and a halide as a secondary component. The disclosed process utilizes synthesis gas ($H_2$+CO), so the process is economical in respect of a raw material. However in the process of the patent publication since a carbonylation reaction by carbon monoxide and a reduction reaction by hydrogen occur at the same time, by-products, such as ethyl acetate and propionic acid are likely to be formed. Judging from the stoichiometric equation, acetic acid is formed as a secondary product. Therefore, the yield of ethylidenediacetate is unsatisfactory in the process of the patent publication.

EP—$A_3$—0025702 relates to a process for producing ethylidene diacetate which comprises reacting methyl acetate or dimethyl ether with carbon monoxide and hydrogen in the presence of a catalyst comprising nickel and/or cobalt and an iodide and/or bromide under substantially anhydrous conditions. This process is superior in that the catalyst is inexpensive, in comparison with FR—A—2303788 described above. However, it has drawbacks similar to those in FR—A—2303788, deriving from the use of the same raw materials as in FR—A—2303788. Namely, in the practice of this process acetic acid is simultaneously produced, and ethyl acetate and propionic acid are likely to be formed as by-products.

FR—A—2404618 relates to a process for producing carboxylic acids and/or esters thereof which comprises reacting alcohols or ethers with carbon monoxide in the presence of nickel or a nickel compound, iodine or an iodine compound and an organic trivalent-nitrogen-group containing compound. Although this invention includes the reaction of the ether and carbon monoxide to produce a monoester, this reaction is clearly different from that of the present invention in which carbon monoxide is reacted with both acetaldehyde and dimethyl ether to produce ethylidene diacetate. Therefore, the objects of this patent and the present invention are different from each other.

The present inventors carried out research to find a process for synthesizing ethylidenediacetate in a high yield. As a result, we found that ethylidenediacetate can be synthesized by reacting carbon monoxide with at least one compound or mixture of two compounds selected from the group consisting of (1) dimethyl acetal, (2) acetaldehyde and methyl acetate, and (3) acetaldehyde and dimethyl ether in the presence of a specific catalyst.

An object of this invention is to provide a process for synthesizing ethylidenediacetate with little or no by-products.

This invention relates to a process for producing ethylidenediacetate which comprises reacting carbon monoxide with at least one compound or mixture of two compounds selected from the group consisting of (1) dimethyl acetal, (2) acetaldehyde and methyl acetate, or (3) acetaldehyde and dimethyl ether in the presence of a catalyst comprising (a) as a main component at least one material selected from metals belonging to Group VIII of the Periodic Table, compounds of the metals or mixtures thereof and (b) as a secondary component at least one material selected from iodides, bromides or mixtures thereof, and at least one promoter under substantially anhydrous conditions.

The mechanism of the carbonylation reaction of this invention is not perfectly clear. However, it is believed that the reaction is expressed by the following equation:

(1) Reaction of dimethyl acetal and carbon monoxide

$$CH_3CH(OCH_3)_2+2CO \rightarrow CH_3CH(OCOCH_3)_2$$

(2) Reaction of acetaldehyde, methyl acetate and carbon monoxide

$$CH_3CHO+CH_3COOCH_3+CO \rightarrow CH_3CH(OCOCH_3)_2$$

(3) Reaction of acetaldehyde dimethyl ether and carbon monoxide

$$CH_3CHO+CH_3OCH_3+2CO \rightarrow CH_3CH(OCOCH_3)_2$$

In the case of practicing this invention, ethylidenediacetate may be synthesized by one of the three equations. For example, ethylidenediacetate can be synthesized from (1) dimethyl acetal and carbon monoxide, (2) acetaldehyde, methyl acetate and carbonmonoxide or (3) acetaldehyde, dimethyl ether and carbon monoxide. Alternatively, ethylidenediacetate may simultaneously be synthesized by two or three equations as mentioned above. For example, ethylidenediacetate can be synthesized

from (a) acetaldehyde, methyl acetate, dimethyl ether and carbon monoxide by equations (2) and (3); (b) dimethyl acetal, acetaldehyde, methyl acetate or dimethyl ether and carbon monoxide by equations (1) and (2), or (1) and (3); and (c) dimethyl acetal, acetaldehyde, methyl acetate, dimethyl ether and carbon monoxide by equations (1), (2) and (3).

In the synthesis of ethylidenediacetate according to this invention, at least one material selected from metals belonging to Group VIII of the Periodic Table, compounds of the metals or mixtures thereof is used as a main component constituting the catalyst, and at least one material selected from iodides, bromides and mixtures thereof is used as a secondary component constituting the catalyst.

The main components include, for example,

$RhX_3$,
$RhX_3 \cdot 3H_2O$,
$[RhX(CO)_2]_2$,
$Rh_6(CO)_{16}$,
$RhX(PPh_3)_3$,
$Rh(SnCl_3)(PPh_3)_3$,
$RhI(CO)(SbPh_3)_2$,
$RhH(CO)(PPh_3)_3$,
$Rh(AcAc)_3$,
$R(AcO)(PPH_3)_2$,
Metallic Rh,
$Rh_2(CO)_3$,
$RhX(CO)(PPh_3)_2$,
$RhCl(CO)(AsPh_3)_2$,
$RhX(CO)[P(n\text{-}C_4H_9)_3]_2$,
$[Rh(C_2H_4)_2Cl]_2$,
$[Rh(AcO)_2]_2$,
$RhCl(CO)[P(OPh)_3]_2$,
$Rh[P(OPh)_3]_{42}$,
$RhCl(PPh_3)_2(CH_3I)_2$,
$[Rh(CO)_2X][(n\text{-}C_4H_9)_4N]$,
$[Rh_2O_2X_4][(n\text{-}C_4H_9)_4As]_2$,
$[Rh(CO)I_4][(n\text{-}C_4H_9)_4P]$,
$K_4Rh_2X_2(SnX_3)_4$,
$IrX_3$,
$IrX_3 \cdot 3H_2O$,
$Ir_2(CO)_4X_2$,
$Ir_2(CO)_8$, $Ir(PPh_3)_2(CO)X$,
$Ir(PPh_3)_2(CH_3I)_2$,
$Ir(SnCl_3)(PPh_3)_3$,
$IrCl(CO)(AsPh_3)_2$,
$IrI(CO)(SbPh_3)_2$,
$[Ir(CO)_2X_2][(n\text{-}C_4H_9)_4n]$,
$[Ir_2(CO)_2X_4][(n\text{-}C_4H_9)_4As]$,
$[Ir(CO)_2I_4][(n\text{-}C_4H_9)_4P]$,
$Ir(PPh_3)_2(CO)Br$,
$Ir[(n\text{-}C_4H_9)_3P]_2(CO)Br$,
$Ir[(n\text{-}C_4H_9)_3P]_2(CO)I$,
$IrX(PPh_3)_3$,
$IrCl(PPh_3)_3H(CO)$,
$[Ir(C_2H_4)_2Cl]_2$,
$K_4Ir_2X_2(SnX_3)_4$,
$PdX_2$,
$Pd(OAc)_2$,
$[Pd(CO)X_2]_2$,
$[Pd(PPh_3)_2]X_2$,
$Ir[(n\text{-}C_4H_9)_3P]_2(CO)I$,
$IrX(PPh_3)_3$,
$IrCl(PPh_3)_3H(CO)$,
$[Ir(C_2H_4)_2Cl]_2$,
$K_4Ir_2X_2(SnX_3)_4$,
$PdX_2$,
$[Pd(CO)X_2]_2$,
$[Pd(RPh_3)_2]X_2$,
$[Pd(PPh_3)]_2(CO)Br$,
$(PdX_4)[(n\text{-}C_4H_9)_4P]$,
$Pd[n\text{-}C_4H_9)_3P](CO)Cl_2$,
$PdCl(PPh_3)_2(SnCl_3)$,
$Pd[(n\text{-}C_4H_9)_3P]_2I_2$,
$RuX_3$,
$RuX_3 \cdot 3H_2O$,
$Ru(CO)_2I_2$,
$Ru(CO)_{12}$,
$RuCl_2(CO)(PPh_3)_3$,
$RuI_2(CO)(AsPh_3)_3$,
$RuBr_2(CO)[(n\text{-}C_4H_9)_3P]_3$,
$RuBr_3(CO)(PPh_3)_2$,
$K_2[Ru_2(SnCl_3)_2]$,
$H_2PtX_6$,
$Pt(CO)X_2$,
$Pt(AsPh_3)(CO)I_2$,
$Pt[AsPh_3]_2$,
$[Pt(CO)X_3][(n\text{-}C_4H_9)_4N]$,
$Pt(PPh_3)_2CH_3I$,
$[PtX_4][(n\text{-}C_4H_9)_4As]$,
$Pt(PPh_3)_2(SnBr_3)_2$,
$PtCl(AsPh_3)_2(SnCl_3)_2$,
$Pt[P(C_2H_5)_3]_2CH_3I$,
$Os(CO)_4X_2$,
$Os_3(CO)_{12}$,
$Os(CO)_5$,
$OsCl_3(CO)[(n\text{-}C_4H_9)_3P]_2$,
$OsBr_3(AsPh_3)_3$,
$OsBr_3(AsPh_3)_3$,
$OsBr(PPh_3)_3$,
Metallic Ni,
$NiX_2$,
$NiX_2 \cdot 3H_2O$,
$Ni(CO)_4$,
$Ni(CO)_2(PPh_3)_2$,
$Ni(AcAc)_2$,
$CoI_2$,
$[Co(CO)_4]_2$,
$HCo(CO)_4$,
$FeI_2$,
$Fe(CO)_5$,
$H_2Fe(CO)_4$,

wherein X is F, Cl, Br or I, Ph is phenyl group, OPh is phenoxy group, AcO is acetoxy group and AcAc is acetylacetonate group.

Of these metals and metal compounds, palladium, iridium, ruthenium, platinum rhodium, nickel and cobalt, and compounds of the metals are preferable as a main component with palladium, rhodium, nickel and cobalt and their compounds of the metals being more preferred; palladium, rhodium and nickel and their compounds are most preferred. Any of the above metals or their compounds may be used alone or as a mixture.

An iodide, a bromide and mixture thereof may be used as a secondary component. An

iodide is preferred. Iodides and/or bromide being generally present in the reaction a medium in the form of an alkyl halide, such as methyl iodide, an acid halogenide, such as acetyl iodide or hydrogen halide, such as hydrogen iodide may be used as a secondary component. The secondary component may be added to a reaction medium as it is. Materials which can convert to an alkyl halide, an acid halogenide or hydrogen halide can be used as a secondary component. Examples of the materials which convert to an alkyl halide, an acid halogenide or a hydrogen halide by reacting with components in the reaction medium include inorganic halides, such as alkali metal halides, and alkaline earth metal halides, iodine and bromine.

While the carbonylation reaction of this invention proceeds satisfactorily in the presence of a catalyst containing the above mentioned main component and the above-mentioned secondary component, an organic promoter or an inorganic promoter is also used with the catalyst in order to increase the reaction rate. An organic promoter is preferable. The organic promoter may be introduced into the reaction system with the reactants.

The organic promoters are organic nitrogen, phosphorus, antimony and arsenic compounds. The compounds represented by the following formula are included in the above organic promoters:

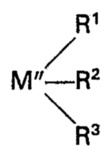

wherein M″ is N, P, Sb or As and $R^1$, $R^2$ and $R^3$ may be the same or different, and independently hydrogen, or alkyl having 1—10 carbon atoms, cycloalkyl having 3—10 carbon atoms or aryl having 7—10 carbon atoms. Examples of the promoters include amines, such as monomethyl amine, dimethyl amine, trimethyl amine, dimethyl ethyl amine, diethyl amine, tri-iso-propyl amine, tri-n-propyl amine, tri-n-butylamine, tri-tert.-butylamine, aniline, dimethyl aniline, diethyl aniline and the like; phosphines, such as tri-n-propyl phosphine, tri-iso-propyl phosphine, tri-n-butyl phosphine, tri-tert.-butyl phosphine, tricyclohexyl phosphine, ethylene bis(diphenyl phosphine), triphenyl phosphine and the like; arsines, such as trimethyl arsine, triethyl arsine, tri-iso-propyl arsine, tripropyl arsine, tricyclohexyl arsine, phenyl di-iso-propyl arsine, diphenyl arsine, bis(diphenyl arsino)ethane, bis(di-iso-propyl arsino)hexane, and the like; and stibines, such as tri-iso-propyl stibine, ethyl-di-iso-propyl stibine, triphenyl stibine, tri(o-tolyl)stibine, phenyl diamyl stibine, tris(diethyl aminomethyl)stibine, bis(diethylstibino)pentane and the like.

In addition to the above listed compounds, organic nitrogen compounds and organic compounds having oxygen or phosphorus atoms and nitrogen atoms may be used as an organic promoter for the reaction.

Examples of the organic nitrogen compounds include heterocyclic compounds such as pyrrole, pyrrolidine, piperidine, pyrimidine, picolines, pyrazine and N-alkyl ($C_1$—$C_5$) substituted derivatives of the above mentioned compounds such as N-methylpyrrolidine, benzotriazole, piperazine, N-methyl piperazine, N-ethyl piperazine, 2-methyl-N-methylpiperazine, 2,2-dipyridyl, methyl-substituted 2,2-dipyridyl, 1,4-diazabicyclo(2,2,2) octane, methyl-substituted 1,4-diazabicyclo(2,2,2)octane, purine, 2-aminopyridine, 1,10-phenanthroline, methyl-substituted 1,10-phenanthroline, 2-(dimethylamino)-pyridine, 2-(dimethlamino)-6-methoxyquinoline, 7-chloro-1,10-phenanthroline 4-triethylsilyl-2,2′-dipyridyl, 5-(thiabenzyl)-1,10-phenanthroline, pyridine, 2,4-dimethyl pyridine, 2,6-dimethyl pyridine, 2,4,6-trimethylpyridine, imidazole and the like; diamines, such as N,N,N′,N′-tetramethyl ethylene diamine, N,N,N′,N′-tetraethyl ethylene diamine, N,N,N′,N′-tetra-n-propyl ethylene diamine, N,N,N′,N′-tetramethyl methylene diamine, N,N,N′,N′-tetraethyl methylene diamine, N,N,N′,N′-tetra-iso-butyl methylene diamine, and the like; and nitriles, such as acetonitrile, propionitrile, adiponitrile, benzonitrile and the like.

The organic compounds having oxygen or phosphorus atoms and nitrogen atoms include hydroxy or carboxyl-substituted above mentioned organic nitrogen compound, such as 2-hydroxypyridine, methyl-substituted 2-hydroxypyridine, picolinic acid, methyl-substituted picolinic acid, 2,5-dicarboxy piperazine, ethylenediamine tetraacetic acid, 2,6-dicarboxy pyridine, 8-hydroxy quinoline, 2-carboxyquinoline, cyclohexane-1,2-diamine-N,N,N′,N′-tetraacetic acid, salts of ethylenediamine tetraacetic acid, tetramethyl ester of ethylenediamine tetraacetic acid, ammonium salts like ammonium acetate; carboxylic amides, such as acetamide, acetanilide, N,N-dimethyl acetamide, N-methyl-N-phenyl acetamide and the like; amino acid, such as N,N-dimethyl glycine, N,N-diethyl glycine and the like; 1-methyl-2-pyrrolidone, 4-methyl morpholine N,N,N′,N′-tetramethyl urea, N-methyl iminodiacetic acid, nitrilotriacetic acid, N-methyl iminodiacetic acid, phosphine, iminium salts such as triphenyl phosphine iminium chloride. Of these promoters, organic promoters having trivalent phosphous and trivalent nitrogen are particularly preferred.

The amount of metal belonging to Group VIII of the Periodic Table employed as the main component may be in the range of from $10^{-6}$ to 5 mol, preferably $10^{-4}$—1 mol and more preferably $10^{-3}$ to 0.5 mol, per liter of total amount of liquid starting material(s) and solvent. The amount of iodide component and/or bromide

component employed as secondary component may be in the range of from $10^{-6}$ to 15 mol, preferably $10^{-5}$ to 5 mol and more preferably $10^{-4}$ to 3 mol per liter of total amount of liquid starting material(s) and solvent.

The amount of the organic or inorganic promoter employed depends on the amount of main catalyst employed. In general, the amount of the organic or inorganic promoter employed may be in the range of from $10^{-6}$ to 10 mol, preferably $10^{-4}$ to 5 mol per liter of total amount of starting material(s) and solvent.

In practicing this invention, the reaction temperature is not critical. In general, the reaction temperature may be in the range of 20°C—500°C, preferably 30—350°C, more preferably 40—250°C.

The reaction pressure is kept high enough to keep the raw material(s), the solvent and the product in a liquid phase and to maintain appropriately partial pressure of carbon monoxide. The partial pressure of carbon monoxide may be in the range of 0.5—350 atm, preferably 1—300 atm, and more preferably 3—250 atm. However, partial pressure of carbon monoxide may be in the range of 0.05—1000 atm.

Carbon monoxide used as a starting material need not necessarily have high purity. The carbon monoxide may contain hydrogen, carbon dioxide, methane, nitrogen and rare gases. Hydrogen contained in the carbon monoxide tends to make the catalyst stable and assists to proceed to reaction effectively. However, carbon monoxide of extremely low purity is not preferred, because it increases the pressure of the reaction system.

Dimethyl acetal, acetaldehyde, methyl acetate and dimethyl ether can be produced by processes which do not use petrochemicals such as ethylene, etc. as a starting material for they can be synthesized from methanol and synthesis gas ($H_2$+CO) [refer to Patent Publication (for opposition) No. 2525/1973, Patent Publication (laid open) No. 149213/1976, Patent Publication (laid open) No. 136110/1977 and Patent Publication (laid open) No. 136111/1977]. The starting materials produced by the above processes may contain impurities such as methanol, acetic acid etc. formed as by-products, and such impurities can be tolerated.

In the case of synthesizing ethylidenediacetate from acetaldehyde, methyl acetate or dimethyl ether and carbon monoxide, the molar ratio of acetaldehyde to methyl acetate or dimethyl ether may vary over a wide range. But, in order to increase the conversion ratio of acetaldehyde, methyl acetate or dimethyl ether is used in excess of the stoichimetric ratio.

The product, ethylidenediacetate, produced according to this invention is likely to decompose in the presence of water. After the raw material and the solvent have been dried to a substantially anhydrous state, they should be introduced into the reaction system. The reaction system should be kept in a substantially anhydrous state. In other words, water content in the reaction system should be kept to less than 10 mol% on the basis of total mol of the liquid components present in the reaction system, preferably less than 5 mol% and more preferably less than 3 mol%.

When methyl acetate or dimethyl ether either is used as a starting material, it serves as a solvent for reaction. So another solvent is not necessary. However, when dimethyl acetal is used as a starting material or even when methyl acetate or dimethyl ether is used as a starting material, an organic solvent and a diluent compatible with the raw materials and the product under the reaction conditions may be used. Examples of the organic solvent include organic acids, such as acetic acid, propionic acid, butyric acid, octanoic acid, phthalic acid, benzoic acid; esters of organic acids, such as methyl acetate, ethyl acetate, ethylene glycol diacetate, propylene glycol diacetate, dimethyl adipate, methyl benzoate, ethyl benzoate, dimethyl phthalate, diethyl phthalate, dioctyl phthalate, phenyl acetate, and tolyl acetate, hydrocarbons, such as dodecane, hexadecane, benzene, naphthalene, and biphenyl; esters of inorganic acids, such as triphenyl phosphate, tricresyl phosphate, dibutylphenyl phosphate, silicates such as tetramethyl ortho-silicate, and tetrabutyl silicate; aromatic ethers, such as diphenyl ethers; ketones, such as acetone, methyl ethyl ketone, dibutyl ketone, methyl isobutyl ketone, acetophenone, and benzophenone.

When dimethyl acetal or dimethyl ether is used as a starting material, acetic acid or its derivative, for example, methyl acetate, other carboxylic esters, carboxylic anhydride, such acetic anhydride, or mixtures thereof may be used as a solvent. When methyl acetate is used as a starting material, methyl acetate itself, acetic acid which is a derivative of methyl acetate or mixture thereof may be used as a solvent. These solvents make the reaction system and the catalyst system stable and increase selectivity and yield of the product.

The present process may be carried out by batch, semicontinuous or continuous method. When the semi-continuous and continuous methods are used, the concentration of acetaldehyde or dimethyl acetal can be kept at such a lower level that little or no polymerization of acetaldehyde or dimethyl acetal occurs, so these methods are preferable. The continuous method is more preferable from an industrial view point.

This invention is further illustrated by nonlimiting Examples.

Example 1

Into a reactor were charged 0.341 grs. of rhodium trichloride, 1.36 grs. of triphenyl phosphine, 28.4 grs. of methyl iodide and 60 ml of methyl acetate (solvent). Air in the reactor was

purged with carbon monoxide. The inside of the reactor was superpressurized and then was heated to 150°C and was maintained at this temperature. 9.0 Grs. of dimethyl acetal under pressure was continuously charged over 2.6 hrs. into the reactor which was maintained at a total pressure of 90 Kg/cm$^2$ (CO partial pressure of 80 Kg/cm$^2$). After charging of the starting materials was completed, the reaction was continued for 1.5 hours. After cooling the reaction mixture, GC analysis showed that it contained 11.0 grs. of ethylidenediacetate. The theoretical yield of the product was 75.3 mol% on the basis of dimethyl acetal.

Example 2

Into a reactor were charged 0.35 grs. of chlorocarbonyl bis (triphenylphosphine)rhodium, 2.0 grs. of lithium acetate, 14.2 grs. of methyl iodide and 60 ml of methyl acetate (solvent). Air in the reactor was purged with nitrogen. The inside of the reactor was superpressurized and then was heated to 165°C and was maintained at this temperature. 9.0 Grs. of dimethyl acetal under pressure was continuously charged over 3 hours into the reactor which was maintained at a total pressure of 50 Kg/cm$^2$ (CO partial pressure of 40 Kg/cm$^2$). After charging of the starting materials was completed, the reaction was continued for 2 hours. After cooling the reaction mixture, GC analysis showed that it contained 10.4 grs. of ethylidenediacetate. The theoretical yield of the product was 71.2 mol% on the basis of dimethyl acetal.

Example 3

Into a reactor were charged 1.0 gr. of palladium acetate, 5.0 grs. of triphenylphosphine, 25.5 grs. of methyl iodide and 60 ml of methyl acetate (solvent). Air in the reactor was purged with nitrogen. The inside of the reactor was superpressurized and then was heated to 150°C and was maintained at this temperature. 9.0 grs. of dimethyl acetal under pressure was charged over 2 hours into the reactor which was maintained at a total pressure of 100 Kg/cm$^2$ (CO partial pressure of 90 Kg/cm$^2$). After charging of the starting materials was completed, the reaction was continued for 2 hours. After cooling the reaction mixture, GC showed that it contained 6.6 grs. of ethylidenediacetate. The theoretical yield of the product was 45.2 mol% on the basis of dimethyl acetal.

Example 4

Into a reactor were charged 2.57 grs. of nickel acetylacetone, 4.1 grs. of tri(n-butyl)phosphine, 21.3 grs. of methyl iodide and 30 ml of methyl acetate and 50 ml of acetic acid (solvent). Air in the reactor was purged with nitrogen. The inside of the reactor was superpressurized and then was heated to 175°C and was maintained at this temperature. 9.0 grs. of dimethyl acetal under pressure was continuously charged over 3 hours into the reactor which was maintained at a total pressure of 53 Kg/cm$^2$ (CO partial pressure of 40 Kg/cm$^2$). After charging of the starting materials was completed, the reaction was continued for 2 hours. After cooling the reaction mixture, GC analysis showed that it contained 5.9 grs. of ethylidenediacetate. The theoretical yield of the product was 40.4 mol% on the basis of dimethyl acetal.

Example 5

Into a reactor were charged 0.341 grs. of rhodium trichloride, 0.56 grs. of 2,6-lutidine, 21.3 grs of methyl iodide and 30 ml methyl acetate and 50 ml of acetic acid (solvent). Air in the reactor was purged with nitrogen. The inside of the reactor was superpressurized and then was heated to 165°C and was maintained at this temperature. 9.0 grs. of dimethyl acetal under pressure was charged over 3 hours into the reactor which was maintained at a total pressure of 70 Kg/cm$^2$ (CO partial pressure of 60 Kg/cm$^2$). After charging of the starting materials was completed, the reaction was continued for 2 hours. After cooling the reaction mixture, GC analysis showed that it contained 9.8 grs. of ethylidenediacetate. The theoretical yield of the product was 67.1 mol% on the basis of dimethyl acetal.

Example 6

Into a reactor were charged 0.341 grs. of rhodium trichloride, 1.36 grs. of triphenyl phosphine, 28.4 grs. of methyl iodide and 60 ml of methyl acetate (Sol+S.M.). Air in the reactor was purged with carbon monoxide. The inside of the reactor was superpressurized and then was heated to 150°C and was maintained at this temperature. 8.8. grs. of acetaldehyde and 14.8 grs. of methyl acetate under pressure were continuously charged over 2 hours into the reactor which was maintained at a total pressure of 90 Kg/cm$^2$ (CO partial pressure of 80 Kg/cm$^2$). After charging of the starting materials was completed, the reaction was continued for 2 hours. After cooling the reaction mixture, GC analysis showed that it contained 21.8 grs. of ethylidenediacete. The theoretical yield of the product was 74.7 mol% on the basis of acetaldehyde.

Example 7

Into a reactor were charged 0.35 grs. of chlorocarbonyl bis(triphenylphosphine)rhodium, 2.0 grs. of lithium acetate, 14.2 grs of methyl iodide and 60 ml of methyl acetate (Sol+S.M.). Air in the reactor was purged with nitrogen. The inside of the reactor was superpressurized and then was heated to 165°C and was maintained at this temperature. 8.8 grs. of acetaldehyde and 14.8 grs. of methyl acetate under pressure were continuously charged over 2 hours into the reactor which was maintained at a total pressure of 50 Kg/cm$^2$ (CO partial

pressure of 40 $Kg/cm^2$). After charging of the starting materials was completed, the reaction was continued for 2 hours. After cooling the reaction mixture, GC analysis showed that it contained 19.6 grs. of ethylidenediacetate. The theoretical yield of the product was 67.1 mol% on the basis of acetaldehyde.

Example 8

Into a reactor were charged 1.0 gr. of palladium acetate, 5.0 grs. of triphenylphosphine, 25.5 grs. of methyl iodide and 60 ml of methyl acetate (Sol+S.M.). Air in the reactor was purged with nitrogen. The inside of the reactor was superpressurized and then was heated to 150°C and was maintained at this temperature. 8.8 grs. of acetaldehyde and 14.8 grs. of methyl acetate under pressure were charged over 2 hours into the reactor which was maintained at a total pressure of 100 $Kg/cm^2$ (CO partial pressure of 90 $Kg/cm^2$). After charging of the starting materials was completed, the reaction was continued for 2 hours. After cooling the reaction mixture, GC showed that it contained 14.1 grs. of ethylidenediacetate. The theoretical yield of the product was 48.3 mol% on the basis of acetaldehyde.

Example 9

Into a reactor were charged 2.57 grs. of nickel acetylacetone, 4.1 grs. of tri(n-butyl)phosphine, 21.3 grs. of methyl iodide and 30 ml of methyl acetate and 50 ml of acetic acid (Sol+S.M.). Air in the reactor was purged with nitrogen. The inside of the reactor was superpressurized and then was heated to 175°C and was maintained at this temperature. 8.8 grs. of acetaldehyde and 14.8 grs. of methyl acetate under pressure were continuously charged over 2 hours into the reactor which was maintained at a total pressure of 53 $Kg/cm^2$ (CO partial pressure of 40 $Kg/cm^2$). After charging of the starting materials was completed, the reaction was continued for 2 hours. After cooling the reaction mixture, GC analysis showed that it contained 15.0 grs. of ethylidenediacetate. The theoretical yield of the product was 51.4 mol% on the basis of acetaldehyde.

Example 10

Into a reactor were charged 0.341 grs. of rhodium trichloride, 0.56 grs. of 2.6-lutidine, 21.3 grs. of methyl iodide and 30 ml of methyl acetate and 50 ml of acetic acid (Sol+S.M.). Air in the reactor was purged with nitrogen. The inside of the reactor was superpressurized and then was heated to 165°C and was maintained at this temperature. 8.8 Grs. of acetaldehyde and 14.8 grs. of methyl acetate under pressure were charged over 2 hours into the reactor which was maintained at a total pressure of 70 $Kg/cm^2$ (CO partial pressure of 60 $Kg/cm^2$). After charging of the starting materials was completed, the reaction was continued for 2 hours. After cooling the reaction mixture, GC analysis showed that it contained 19.1 grs. of ethylidenediacetate. The theoretical yield of the product was 65.4 mol% on the basis of acetaldehyde.

Note: GC means gas chromatography.

Sol+S.M. means "as solvent and starting material".

## Claims

1. A process for producing ethylidenediacetate which is characterised by reacting carbon monoxide with at least one compound or mixture of two compounds selected from (1) dimethyl acetal, (2) acetaldehyde and methyl acetate, and (3) acetaldehyde and dimethyl ether in the presence of a catalyst comprising (a) as a main component at least one material selected from metals belonging to Group VIII of the Periodic Table, compounds of the metals and mixtures thereof and (b) as a secondary component at least one material selected from iodides, bromides and mixtures thereof, and at least one promoter under substantially anhydrous conditions, the promoter being selected from organic nitrogen compounds, organic phosphorous compounds, organic antimony compounds and organic arsenic compounds.

2. A process as claimed in claim 1 characterised in that the main component constituting the catalyst is selected from palladium, rhodium, nickel, cobalt and compounds of these metals.

3. A process as claimed in claim 1 or 2 characterised in that the secondary component is at least one iodide.

4. A process as claimed in any one of claims 1 to 3 characterised in that the amount of the main component is in the range of from $10^{-6}$ to 5 mol per liter of the total amount of liquid starting material(s) and solvent.

5. A process as claimed in any one of claims 1 to 4 characterised in that the amount of the secondary component is in the range of from $10^{-6}$ to 15 mol per liter of the total amount of liquid starting material(s) and solvent.

6. A process as claimed in any one of claims 1 to 5 characterised in that the amount of the promoter employed is in the range of from $10^{-6}$ to 10 mol per liter of the total amount of starting material(s) and solvent.

7. A process as claimed in any one of claims 1 to 6 characterised in that the reaction pressure is kept high enough to keep the raw material and the product and optional solvent in a liquid phase.

8. A process as claimed in any one of claims 1 to 7 characterised in that the reaction is effected at a temperature in the range of from 20°C to 500°C.

**Patentansprüche**

1. Verfahren zur Herstellung von Ethylidendiacetat, dadurch gekennzeichnet, daß man Kohlenmonoxid mit mindestens einer Verbindung oder einer Mischung aus zwei Verbindungen, bestehend aus

(1) Dimethylacetal,
(2) Acetaldehyd und Methylacetat und
(3) Acetaldehyd und Dimethylether,

in Gegenwart eines Katalysators aus

(a) mindestens einem Metall der Gruppe VIII des Periodensystems und/oder einer Verbindung dieser Metalle als Hauptbestandteil und
(b) mindestens einem Jodid und/oder Bromid als zweitem Bestandteil

sowie in Gegenwart mindestens eines aus einer organischen Stickstoff-, Phosphor-, Antimon- oder Arsenverbindung bestehenden Beschleunigers unter praktisch wasserfreien Bedingungen umsetzt.

2. Verfahren nach Anspruch 1, dadurch gekennzeichnet, daß man einen Katalysator verwndet, dessen Hauptbestandteil aus Palladium, Rhodium, Nickel, Kobalt oder einer Verbindung dieser Metalle besteht.

3. Verfahren nach Ansprüchen 1 oder 2, dadurch gekennzeichnet, daß man einen Katalysator verwendet, dessen zweiter Bestandteil aus mindestens einem Jodid besteht.

4. Verfahren nach einem der Ansprüche 1 bis 3, dadurch gekennzeichnet, daß man pro Liter der Gesamtmenge an flüssigem (flüssigen) Ausgangsmaterial(ien) und Lösungsmittel $10^{-6}$ bis 5 Mol(e) Katalysatorhauptbestandteil verwendet.

5. Verfahren nach einem der Ansprüche 1 bis 4, dadurch gekennzeichnet, daß man pro Liter der Gesamtmenge an flüssigem (flüssigen) Ausgangsmaterial(ien) und Lösungsmittel $10^{-6}$ bis 15 Mol(e) zweiten (Katalysator)-Bestandteil verwendet.

6. Verfahren nach einem der Ansprüche 1 bis 5, dadurch gekennzeichnet, daß man pro Liter der Gesamtmenge an Ausgangsmaterial(ien) und Lösungsmittel $10^{-6}$ bis 10 Mol(e) Beschleuniger verwendet.

7. Verfahren nach einem der Ansprüche 1 bis 6, dadurch gekennzeichnet, daß man den Reaktionsdruck hoch genug wählt, um das Ausgangsmaterial, das Produkt und gegebenenfalls das Lösungsmittel in flüssiger Phase zu halten.

8. Verfahren nach einem der Ansprüche 1 bis 7, dadurch gekennzeichnet, daß man die Umsetzung bei einer Temperatur im Bereich von 20—500°C durchführt.

**Revendications**

1. Procédé de production d'éthylidène diacétate qui est caractérisé par la réaction d'oxyde de carbone avec au moins un composé ou un mélange de deux composés choisis parmi (1) le diméthyl acétal, (2) l'acétaldéhyde et l'acétate de méthyle et (3) l'acétaldéhyde et le diméthyl éther en présence d'un catalyseur comprenant (a), comme composant principal, au moins un matériau choisi parmi les métaux appartenant au groupe VIII de la Table Périodique, composés des métaux et leurs mélanges et (b) comme composant secondaire, au moins un matériau choisi parmi des iodures, bromures et leurs mélanges, et au moins un agent d'activation en conditions sensiblement anhydres, l'agent d'activation étant choisi parmi des composés organiques d'azote, des composés organiques, phosphoreux, des composés organiques d'antimoine et des composés organiques d'arsenic.

2. Procédé selon la revendication 1, caractérisé en ce que le composant principal constituant le catalyseur est choisi parmi le palladium, le rhodium, le nickel, le cobalt et des composés de ces métaux.

3. Procédé selon la revendication 1 ou 2, caractérisé en ce que le composant secondaire est au moins un iodure.

4. Procédé selon l'une quelconque des revendications 1 à 3, caractérisé en ce que la quantité du composant principal est comprise entre $10^{-6}$ et 5 moles par litre de la quantité totale de la matière ou des matières premières liquides et du solvant.

5. Procédé selon l'une quelconque des revendications 1 à 4, caractérisé en ce que la quantité du composant secondaire est comprise entre $10^{-6}$ et 15 moles par litre de la quantité totale de la matière ou des matières premières liquides et du solvant.

6. Procédé selon l'une quelconque des revendications 1 à 5, caractérisé en ce que la quantité de l'agent d'activation employé est comprise entre $10^{-6}$ et 10 moles par litre de la quantité totale de la matière ou des matières premières et du solvant.

7. Procédé selon l'une quelconque des revendications 1 à 6, caractérisé en ce que la pression de la réaction est maintenue suffisamment élevée pour maintenir la matière première et le produit et éventuellement le solvant en phase liquide.

8. Procédé selon l'une quelconque des revendications 1 à 7, caractérisé en ce que la réaction est effectuée à une température comprise entre 20°C et 500°C.